# EUROPÄISCHE PATENTSCHRIFT

(11) **EP 3 233 035 B1**
(45) Veröffentlichungstag und Bekanntmachung des Hinweises auf die Patenterteilung: **01.05.2019**
(21) Anmeldenummer: 15786975.1
(22) Anmeldetag: 29.10.2015
(51) Int. Cl.: A61K 8/37, A61Q 5/06, A61Q 5/10, A61K 8/06, A61K 8/22, A61K 8/34

(54) **VERDICKENDER ENTWICKLER FÜR OXIDATIONSFÄRBEMITTEL**
THICKENING DEVELOPER FOR OXIDATION PRODUCTS
EPAISSISSANT DEVELOPPEUR POUR DES PRODUITS OXIDATIVES

(30) Priorität: 19.12.2014 DE 102014226540
(43) Veröffentlichungstag der Anmeldung: 25.10.2017
(73) Patentinhaber: Henkel AG & Co. KGaA, 40589 Düsseldorf (DE)
(72) Erfinder: WITTE, Christiane, 25491 Hetlingen (DE); SCHWARTZ, Stephan, 22880 Wedel (DE)
(86) Internationale Anmeldenummer: PCT/EP2015/075084
(87) Internationale Veröffentlichungsnummer: WO 2016/096223

(56) Entgegenhaltungen:
- WO-A1-2006/134051
- WO-A1-2012/032671
- DE-A1-102012 223 202
- Anonymous: "GNPD - Cream Colouration Kit", , 1. Oktober 2008 (2008-10-01), XP055231766, Gefunden im Internet: URL:http://www.gnpd.com/sinatra/recordpage /982880/from_search/w0ilOKHxSH/ [gefunden am 2015-11-26]

## Beschreibung

Die vorliegende Anmeldung betrifft Zusammensetzungen, die sich als Oxidationsmittel für emulsionsförmige, insbesondere cremeförmige, Haaraufhell- und/oder Haarfärbezubereitungen eignen, wobei die emulsionsförmigen Haaraufhell- und/oder Haarfärbezubereitungen bevorzugt in Form einer Öl-in-Wasser-Emulsion vorliegen und mindestens ein Alkalisierungsmittel und gegebenenfalls mindestens ein Oxidationsfarbstoffvorprodukt enthalten und einen pH-Wert im Bereich von 8 bis 11,5, gemessen bei 20°C, aufweisen, und mit denen sich die die Anwendungseigenschaften der Anwendungsmischung, wie Dispersionsstabilität und Tropfverhalten der Mischung bzw. Verbleib der Mischung auf dem Haar, sowie die Haut- und Kopfhautverträglichkeit deutlich verbessern lassen. Ein weiterer Gegenstand der vorliegenden Anmeldung betrifft Mittel zur Farbveränderung keratinischer Fasern, die aus zwei voneinander getrennten Zusammensetzungen durch Vermischen der beiden Zusammensetzungen herstellbar sind, wobei eine der beiden Zusammensetzungen eine oxidative Zusammensetzung gemäß dem ersten Gegenstand der Anmeldung und die zweite Zusammensetzung eine Haaraufhell- und/oder Haarfärbezubereitung ist, die in Form einer Öl-in-Wasser-Emulsion vorliegt und mindestens ein Alkalisierungsmittel und gegebenenfalls mindestens ein Oxidationsfarbstoffvorprodukt enthält und einen pH-Wert im Bereich von 8 bis 11,5, gemessen bei 20°C, aufweist und weiterhin bevorzugt, jeweils bezogen auf ihr Gewicht, 50 - 80 Gew.-% Wasser, weiterhin bevorzugt 5 - 20 Gew.-%, besonders bevorzugt 8 - 15 Gew.-% an mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern aus ein- und mehrwertigen C1-C10-Alkanolen und C8-C30-Alkansäuren sowie Mischungen hiervon, weiterhin bevorzugt mindestens ein nichtionisches und/oder anionisches Tensid in einer Gesamtmenge von 1 - 8 Gew.-%, bevorzugt 2 - 6 Gew.-%, enthält.

Ein weiterer Gegenstand der vorliegenden Anmeldung betrifft ein Kit zur oxidativen Farbveränderung keratinischer Fasern, umfassend eine emulsionsförmige, ggf. Farbstoff-haltige, Alkalisierungszubereitung und eine wässrige Wasserstoffperoxidzubereitung, wobei die Wasserstoffperoxidzubereitung dahingehend optimiert ist, dass die gebrauchsfertige Mischung aus Alkslisierungsemulsion und Wasserstoffperoxidzubereitung eine viskose Creme oder Paste mit einer Viskosität im Bereich von 5000 bis 15000 mPas (beispielsweise gemessen bei 20°C mit Haake-Viskosimeter Typ MV2 mit einer Geschwindigkeit von 8 Umdrehungen/ Minute) darstellt, die sich gut auf die aufzuhellenden und/oder zu färbenden Fasern auftragen lässt und dort während der Anwendungsdauer von 5 bis 60 Minuten verbleibt, ohne in größeren Mengen vorzeitig vom Haar abzutropfen.

Ein weiterer Gegenstand der vorliegenden Anmeldung betrifft ein Verfahren zur oxidativen Farbveränderung keratinischer Fasern, wobei das anwendungsbereite Aufhell- und/oder Färbemittel durch Vermischen der Komponenten des vorgenannten Kits unmittelbar vor der Anwendung hergestellt wird, anschließend auf die Fasern, insbesondere Haare, aufgetragen und nach einer Einwirkzeit von 5 bis 60 Minuten wieder abgespült wird.

Die vorliegende Erfindung bezieht sich auf die oxidative Farbveränderung keratinischer Fasern, insbesondere Haaren. Da bei der Behandlung von keratinischer Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen.

Für die Farbveränderung von menschlichen Haaren kennt der Fachmann verschiedene Verfahren. Im allgemeinen werden für die Färbung menschlicher Haare entweder direktziehende Farbstoffe oder Oxidationsfarbstoffe verwendet, welche durch oxidative Kupplung einer oder mehrerer Entwicklerkomponenten untereinander oder mit einer oder mehreren Kupplerkomponenten entstehen. Kuppler- und Entwicklerkomponenten werden auch als Oxidationsfarbstoffvorprodukte bezeichnet. Die mit Oxidationsfarben erzielten Färbungen werden zumeist als permanente oder semipermanente Färbungen bezeichnet.

Als Oxidationsmittel enthalten diese Mittel zumeist Wasserstoffperoxid. Da Wasserstoffperoxid im alkalischen pH-Bereich nur unzureichend lagerstabil ist, bestehen oxidative Färbemittel üblicherweise aus zwei Komponenten, die unmittelbar vor der Anwendung miteinander vermischt werden. Die eine Komponente enthält Wasserstoffperoxid in wässriger Lösung oder Emulsion, wobei diese Zusammensetzung zur Stabilisierung des Wasserstoffperoxids einen sauren pH-Wert im Bereich von 2,5 bis 5,5 aufweist. Die zweite Komponente enthält ein oder mehrere Alkalisierungsmittel in einer solchen Menge, dass die Anwendungsmischung aus beiden Komponenten einen pH-Wert im Bereich von 8 bis 11 aufweist. Sofern die Alkalisierungszubereitung keinen Farbstoff oder nur geringe Mengen an Farbstoff enthält - letzteres dient zur Kaschierung unerwünschter Farbtöne, die bei der Melaninoxidation entstehen können -, handelt es sich um ein Aufhell- oder Bleichmittel. Die Alkalisierungszubereitung kann aber auch Oxidationsfarbstoffvorprodukte und/oder direktziehende Farbstoffe enthalten; die resultierende Anwendungsmischung dient dann als Färbemittel. Daneben gibt es auch Färbekits und Färbeverfahren, bei denen die Anwendungsmischung aus beiden Komponenten einen pH-Wert im Bereich von etwa 6 bis 7,9 aufweist; die Färbeergebnisse dieser so genannten "sauren" Färbungen erreichen allerdings häufig nicht die Güte, die mit alkalischen Anwendungsmischungen erzielt werden.

Zur oxidativen Haarfarbveränderung kommen bevorzugt Alkalisierungszubereitungen zum Einsatz, die in Form einer Öl-in-Wasser-Emulsion vorliegen. Dies begünstigt zum einen die Mischbarkeit mit der wässrigen Oxidationszusammensetzung. Zum anderen trägt der emulgierte Öl- und/oder Fettanteil zur Pflege der Fasern bzw. des Haars sowie zur Verbesserung des Färbeergebnisses bei.

Weiterhin enthalten die Alkalisierungszubereitungen mindestens ein Alkalisierungsmittel und gegebenenfalls mindestens ein Oxidationsfarbstoffvorprodukt und weisen einen pH-Wert im Bereich von 8 bis 11,5, gemessen bei 20°C, auf. Weiterhin werden bevorzugt Alkalisierungszubereitungen eingesetzt, die, jeweils bezogen auf ihr Gewicht, 50 - 80 Gew.-% Wasser, weiterhin bevorzugt 5 - 20 Gew.-%, besonders bevorzugt 8 - 15 Gew.-% mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern von ein- und mehrwertigen C1-C10-Alkanolen mit C8-C30-Alkansäuren sowie Mischungen hiervon, weiterhin bevorzugt mindestens ein nichtionisches und/oder anionisches Tensid in einer Gesamtmenge von 1 - 8 Gew.-%, bevorzugt 2 - 6 Gew.-%, enthalten.

Zur oxidativen Haarfarbveränderung wird üblicherweise die Alkalisierungszubereitung mit einer wässrigen Oxidationsmittelzubereitung vermischt, beispielsweise in einer wiederverschließbaren Flasche oder einem Schüttelbecher, und die daraus resultierende cremeförmige Anwendungsmischung wird auf das zu behandelnde Haar aufgetragen, wo sie für eine Einwirkzeit von 5 bis 60 Minuten verbleibt, bevor sie wieder abgespült wird.

Bei der Entwicklung solcher Produkte muss darauf geachtet werden, dass die Anwendungsmischung eine ausreichend hohe und langzeitstabile Viskosität aufweist, damit sie während der empfohlenen Einwirkzeit auf den Fasern bzw. dem Haar verbleibt und nicht vorzeitig an Viskosität einbüßt und von den Fasern bzw. vom Haar heruntertropft. Andererseits darf die jeweilige Anfangsviskosität der Oxidationszusammensetzung und der Alkalisierungszubereitung nicht zu hoch eingestellt sein, weil es ansonsten schwierig ist, aus beiden Zusammensetzungen eine homogene, klumpenfreie Mischung herzustellen.

Im Stand der Technik sind bereits diverse Lösungsmöglichkeiten für diese Problemstellung bekannt. Zum Beispiel kann die saure Oxidationszusammensetzung größere Mengen (z. B. 1,5 - 5 Gew.-%) eines (Co-)Polymers aus (Meth)acrylsäure, (Meth)acrylsäureestern, (Meth)acrylsäureamiden oder quaternisierten (Meth)acrylsäure-basierten Monomeren enthalten, das beim Vermischen mit der Alkalisierungszubereitung unter dem Einfluss des pH-Wert-Anstiegs stärker verdickt. Da beim Vermischen lokal sehr hohe pH-Werte auftreten können, treten bei Acrylat-verdickten Anwendungsmischungen häufig Inhomogenitäten auf; die inhomogene Verteilung des Färbemittels wiederum beeinträchtigt das Färbeergebnis. Bei Alkalisierungszubereitungen, die Ammoniak enthalten, führt dessen Verdunstung während der Einwirkzeit zu einem Absinken des pH-Werts, wodurch die Anwendungsmischung an Viskosität verliert und im ungünstigsten Fall vom Haar abtropft.

Eine andere Möglichkeit zur Verdickung der Anwendungsmischung ist es, die eine Zusammensetzung (Oxidations- oder Alkalisierungszusammensetzung) mit einem Aniontensid und die andere Zusammensetzung mit einem Kationtensid zu versetzen. Beim Vermischen führt die Wechselwirkung zwischen beiden Tensiden zum gewünschten Anstieg der Viskosität. Allerdings bevorzugen nicht alle Verbraucher die damit erzielte, recht stark pastöse Konsistenz der Anwendungsmischung. Außerdem kann das Kationtensid, insbesondere in Gegenwart von Wasserstoffperoxid, unangenehme Irritationen der Haut oder der Kopfhaut hervorrufen.

Aufgabe der vorliegenden Erfindung war es daher, eine verbesserte Oxidationsmittelzubereitung für oxidative Farbveränderungsmittel, die in Form einer Öl-in-Wasser-Emulsion vorliegen und mindestens ein Alkalisierungsmittel und gegebenenfalls mindestens ein Oxidationsfarbstoffvorprodukt enthalten und einen pH-Wert im Bereich von 8 bis 11,5, gemessen bei 20°C, aufweisen, bereitzustellen, mit denen sich homogene und viskositätsstabile Anwendungsmischungen herstellen lassen, die während der gesamten Einwirkzeit eine ausreichende Viskosität aufweisen und auf dem Haar verbleiben, ohne abzutropfen.

Es wurde überraschend gefunden, dass wässrige Wasserstoffperoxid-Zubereitungen, enthaltend mindestens einen Polyethylenglycolether eines Monoesters von Glycerin und einer C12-C22-Carbonsäure mit 110 - 500 Ethylenoxideinheiten im Molekül, die gestellte Aufgabe in sehr guter Weise lösen.

Ein erster Gegenstand der vorliegenden Erfindung ist daher eine Oxidationszusammensetzung zur oxidativen Haarbehandlung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens einen Polyethylenglycolether eines Monoesters von Glycerin und einer C12-C22-Carbonsäure mit 110 - 500 Ethylenoxideinheiten im Molekül gemäß der nachstehenden Formel (I) 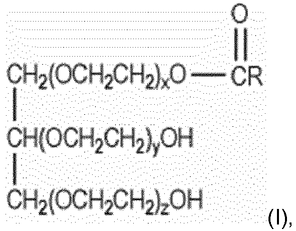 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
wobei alle Mengenangaben auf das Gewicht der Oxidationszusammensetzung bezogen sind.

Die erfindungsgemäße Oxidationszusammensetzung enthält, jeweils bezogen auf ihr Gewicht, 50 - 96 Gew.-%, bevorzugt 70 - 93 Gew.-%, besonders bevorzugt 80 - 90 Gew.-% Wasser.

Die erfindungsgemäße Oxidationszusammensetzung enthält, jeweils bezogen auf ihr Gewicht, 0,5 - 20 Gew.-%, bevorzugt 3 - 12 Gew.-%, besonders bevorzugt 6 - 9 Gew.-% Wasserstoffperoxid. Die erfindungsgemäße Oxidationszusammensetzung enthält mindestens einen Polyethylenglycolether eines Monoesters von Glycerin und einer C12-C22-Carbonsäure mit 110 - 500 Ethylenoxideinheiten im Molekül gemäß der nachstehenden Formel (I) 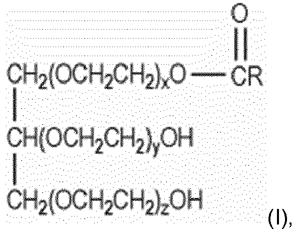 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann.

Erfindungsgemäß bevorzugt ist, dass der Polyethylenglycolether gemäß Formel (I) ausgewählt ist aus Verbindungen, bei denen die Summe der Indizes x + y + z für eine Zahl im Bereich von 120 bis 450, bevorzugt für eine Zahl im Bereich von 150 bis 300, besonders bevorzugt für eine Zahl im Bereich von 200 bis 250, steht.

Der Rest RCO stammt aus einer C12-C22-Carbonsäure, die bevorzugt ausgewählt ist aus Laurinsäure, Myristinsäure, Palmitinsäure, Palmitoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. deren (technischen) Mischungen.

Weiterhin ist es erfindungsgemäß bevorzugt, dass der Polyethylenglycolether gemäß Formel (I) ausgewählt ist aus Verbindungen, bei denen R für einen linearen Alkylrest mit 15 bis 17 C-Atomen steht, der bevorzugt gesättigt ist, steht. Bevorzugt stammt der Rest RCO ab von Palmitinsäure oder Stearinsäure sowie aus Mischungen hiervon.

Erfindungsgemäß besonders bevorzugte Polyethylenglycolether gemäß Formel (I) sind ausgewählt aus PEG-200 Glycerylmonopalmitat, PEG-200 Glycerylmonostearat, PEG-200 Glycerylmonolaurat, PEG-200 Glycerylmonomyristat, PEG-120 Glycerylmonopalmitat, PEG-120 Glycerylmonostearat, PEG-120 Glycerylmonolaurat, PEG-120 Glycerylmonomyristat, PEG-150 Glycerylmonopalmitat, PEG-150 Glycerylmonostearat, PEG-150 Glycerylmonolaurat, PEG-150 Glycerylmonomyristat, PEG-300 Glycerylmonopalmitat, PEG-300 Glycerylmonostearat, PEG-300 Glycerylmonolaurat, PEG-300 Glycerylmonomyristat, sowie Mischungen hiervon. Das ethoxylierte Glycerin kann auch mit Fettsäuren und Fettsäuremischungen verestert sein, die aus nativen Ölen stammen, wie beispielsweise aus Palmöl, Palmkernöl, Kokosöl, Sonnenblumenöl und anderen Ölen. Ein bevorzugtes Beispiel hierfür ist PEG-200 Glycerylmonopalmat. Weiterhin kann es bevorzugt sein, dass das ethoxylierte Glycerin mit hydrogenierten Fettsäuren aus nativen Ölen verestert ist. Ein bevorzugtes Beispiel hierfür ist PEG-200 Hydrogenated Glyceryl Palmate.

Erfindungsgemäß bevorzugte Oxidationszusammensetzungen sind dadurch gekennzeichnet, dass mindestens ein Polyethylenglycolether gemäß Formel (I) in einer Gesamtmenge von 0,05 bis 3 Gew.-%, bevorzugt 0,1 bis 2 Gew.-%, besonders bevorzugt 0,2 - 0,7 Gew.-%, jeweils bezogen auf das Gewicht der Oxidationszusammensetzung, enthalten ist. Weitere erfindungsgemäß bevorzugte Oxidationszusammensetzungen sind dadurch gekennzeichnet, dass mindestens ein Polyethylenglycolether gemäß Formel (I), ausgewählt aus PEG-200 Glycerylmonopalmitat, PEG-200 Glycerylmonostearat, PEG-200 Glycerylmonolaurat, PEG-200 Glycerylmonomyristat, PEG-120 Glycerylmonopalmitat, PEG-120 Glycerylmonostearat, PEG-120 Glycerylmonolaurat, PEG-120 Glycerylmonomyristat, PEG-150 Glycerylmonopalmitat, PEG-150 Glycerylmonostearat, PEG-150 Glycerylmonolaurat, PEG-150 Glycerylmonomyristat, PEG-300 Glycerylmonopalmitat, PEG-300 Glycerylmonostearat, PEG-300 Glycerylmonolaurat, PEG-300 Glycerylmonomyristat, PEG-200 Glycerylmonopalmat, PEG-200 Hydrogenated Glyceryl Palmate sowie Mischungen hiervon, in einer Gesamtmenge von 0,05 bis 3 Gew.-%, bevorzugt 0,1 bis 2 Gew.-%, besonders bevorzugt 0,2 - 0,7 Gew.-%, jeweils bezogen auf das Gewicht der Oxidationszusammensetzung, enthalten ist. Erfindungsgemäß bevorzugte Oxidationszusammensetzungen enthalten mindestens einen Polyethylenglycolether gemäß Formel (I), ausgewählt aus PEG-200 Glycerylmonopalmitat, PEG-200 Glycerylmonostearat, PEG-120 Glycerylmonopalmitat, PEG-120 Glycerylmonostearat, PEG-150 Glycerylmonopalmitat, PEG-150 Glycerylmonostearat, PEG-300 Glycerylmonopalmitat, PEG-300 Glycerylmonostearat, PEG-200 Glycerylmonopalmat, PEG-200 Hydrogenated Glyceryl Palmate sowie Mischungen hiervon, in einer Gesamtmenge von 0,05 bis 3 Gew.-%, bevorzugt 0,1 bis 2 Gew.-%, besonders bevorzugt 0,2 - 0,7 Gew.-%, jeweils bezogen auf das Gewicht der Oxidationszusammensetzung. Erfindungsgemäß besonders bevorzugte Oxidationszusammensetzungen enthalten 0,05 bis 3 Gew.-%, bevorzugt 0,1 bis 2 Gew.-%, besonders bevorzugt 0,2 - 0,7 Gew.-%, PEG-200 Hydrogenated Glyceryl Palmate, jeweils bezogen auf das Gewicht der Oxidationszusammensetzung.

Erfindungsgemäß bevorzugte Oxidationszusammensetzungen sind dadurch gekennzeichnet, dass mindestens ein Polyethylenglycolether eines Monoesters von Glycerin und einer C8-C22-Carbonsäure mit 5 - 10 Ethylenoxideinheiten im Molekül gemäß der nachstehenden Formel (II) enthalten ist, 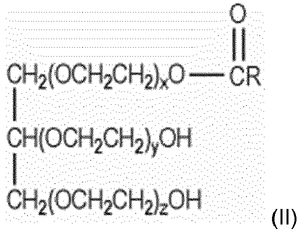 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann. Erfindungsgemäß besonders bevorzugt ist, dass der Polyethylenglycolether gemäß Formel (II) ausgewählt ist aus Verbindungen, bei denen die Summe der Indizes x + y + z für eine Zahl im Bereich von 5 bis 10, bevorzugt für eine Zahl im Bereich von 7 - 9, und R für einen linearen Alkylrest mit 7 bis 17 C-Atomen steht, der bevorzugt gesättigt ist.

Der Rest RCO stammt aus einer C8-C22-Carbonsäure, die bevorzugt ausgewählt ist aus Capronsäure, Caprylsäure, Caprinsäure, Laurinsäure, Myristinsäure, Palmitinsäure, Palmitoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure sowie deren (technischen) Mischungen. Erfindungsgemäß besonders bevorzugte Polyethylenglycolether gemäß Formel (II) sind ausgewählt aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, sowie Mischungen hiervon. Das ethoxylierte Glycerin kann auch mit Fettsäuren und Fettsäuremischungen verestert sein, die aus nativen Ölen stammen, wie beispielsweise aus Kokosöl, Palmöl, Palmkernöl, Sonnenblumenöl und anderen Ölen. Ein bevorzugtes Beispiel hierfür ist PEG-7 Glycerylcocoat. Weiterhin kann es bevorzugt sein, dass das ethoxylierte Glycerin mit hydrogenierten Fettsäuren aus nativen Ölen verestert ist.

Erfindungsgemäß bevorzugte Oxidationszusammensetzungen sind dadurch gekennzeichnet, dass mindestens ein Polyethylenglycolether gemäß Formel (II) in einer Gesamtmenge von 0,03 bis 3 Gew.-%, bevorzugt 0,05 bis 2 Gew.-%, besonders bevorzugt 0,2 - 1,5 Gew.-%, außerordentlich bevorzugt 0,5 bis 1,0 Gew.-%, jeweils bezogen auf das Gewicht der Oxidationszusammensetzung, enthalten ist.

Weitere erfindungsgemäß bevorzugte Oxidationszusammensetzungen sind dadurch gekennzeichnet, dass mindestens ein Polyethylenglycolether gemäß Formel (II), ausgewählt aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, PEG-7 Glycerylcocoat sowie Mischungen hiervon, bevorzugt ausgewählt aus PEG-7 Glycerylcocoat, in einer Gesamtmenge von 0,03 bis 3 Gew.-%, bevorzugt 0,05 bis 2 Gew.-%, besonders bevorzugt 0,2 - 1,5 Gew.-%, außerordentlich bevorzugt 0,5 bis 1,0 Gew.-%, jeweils bezogen auf das Gewicht der Oxidationszusammensetzung, enthalten ist.

Weitere erfindungsgemäß bevorzugte Oxidationszusammensetzungen sind dadurch gekennzeichnet, dass mindestens ein lineares gesättigtes 1-Alkanol mit 12 - 30 Kohlenstoffatomen enthalten ist, bevorzugt in einer Gesamtmenge von 2,7 - 6 Gew.-%, besonders bevorzugt 3,0 bis 5,0 Gew.-%, jeweils bezogen auf das Gewicht der Oxidationszusammensetzung.

Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser Alkanole, besonders bevorzugt aus Cetylalkohol, Stearylalkohol und Cetylalkohol/ Stearylalkohol-Mischungen.

Bevorzugte erfindungsgemäße Oxidationszusammensetzungen enthalten, jeweils bezogen auf ihr Gewicht, mindestens ein lineares gesättigtes 1-Alkanol mit 12 - 30 Kohlenstoffatomen in einer Gesamtmenge von 2,7 - 6 Gew.-%, bevorzugt in einer Gesamtmenge von 3,0 - 5,0 Gew.-%, wobei mindestens ein 1-Alkanol, ausgewählt aus Cetylalkohol, Stearylalkohol und Cetylalkohol/ Stearylalkohol-Mischungen, enthalten ist.

Weitere erfindungsgemäß bevorzugte Oxidationszusammensetzungen sind dadurch gekennzeichnet, dass mindestens ein Polyethylenglycolether eines linearen gesättigten C12-C22-Alkanols mit 15 - 30 Ethylenoxideinheiten im Molekül enthalten ist.

Besonders bevorzugt ist mindestens ein Polyethylenglycolether eines linearen gesättigten C12-C22-Alkanols mit 15 - 30 Ethylenoxideinheiten im Molekül in einer Gesamtmenge von 0,1 - 2 Gew.-%, besonders bevorzugt 0,5 bis 5,0 Gew.-%, jeweils bezogen auf das Gewicht der Oxidationszusammensetzung, enthalten.

Bevorzugt ist der mindestens eine Polyethylenglycolether ausgewählt aus Polyethylenglycolethern von Laurylalkohol, Myristylalkohol und Cetylalkohol, jeweils mit 15 - 30 Ethylenoxideinheiten im Molekül, besonders bevorzugt ausgewählt aus Ceteth-20, Steareth-20, Ceteareth-20, Ceteth-25, Steareth-25, Ceteareth-25, sowie Mischungen hiervon.

Weitere erfindungsgemäß bevorzugte Oxidationszusammensetzungen sind dadurch gekennzeichnet, dass mindestens ein Öl, das bevorzugt ausgewählt ist aus Mineralölen und Paraffinölen, enthalten ist. Besonders bevorzugt ist mindestens ein Öl, das bevorzugt ausgewählt ist aus Mineralölen und Paraffinölen, in einer Gesamtmenge von 0,1 - 5 Gew.-%, besonders bevorzugt 0,2 - 3 Gew.-%, außerordentlich bevorzugt 0,3 - 1 Gew.-%, jeweils bezogen auf das Gewicht der Oxidationszusammensetzung, enthalten.

Erfindungsgemäß bevorzugte Öle sind ausgewählt aus natürlichen und synthetischen Kohlenwasserstoffen, besonders bevorzugt aus Mineralölen, Paraffinölen, C₁₈-C₃₀-Isoparaffinen, insbesondere Isoeicosan, Polyisobutene und Polydecene, die beispielsweise unter der Bezeichnung Emery® 3004, 3006, 3010 oder unter der Bezeichnung Ethylflo® von Albemarle oder Nexbase® 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol®S von BASF).

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol® G 16 S), 2-Hexyldecyllaurat, Isodecylneopentanoat, Isononylisononanoat, 2-Ethylhexylpalmitat (Cegesoft® C 24) und 2-Ethylhexylstearat (Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestertsein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol® CC) oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten Öle einzusetzen.

Die erfindungsgemäße Oxidationszusammensetzung weist bevorzugt eine Viskosität im Bereich von 1500 - 3500 mPas, besonders bevorzugt 2000 - 3000 mPas, auf, jeweils gemessen bei 20°C im Haake-Viskosimeter Typ MV2 mit einer Geschwindigkeit von 8 Umdrehungen/Minute.

Zur Stabilisierung des Oxidationsmittels während der Lagerung ist es insbesondere bevorzugt, wenn die erfindungsgemäße Oxidationszusammensetzung einen sauren pH-Wert aufweist, insbesondere einen pH-Wert im Bereich von 2,5 bis 5,5, bevorzugt von 3,0 bis 5,0. Bevorzugte Acidifizierungsmittel sind Genusssäuren, wie beispielsweise Zitronensäure, Essigsäure, Äpfelsäure oder Weinsäure, sowie verdünnte Mineralsäuren, insbesondere Phosphorsäure.

Zur Stabilisierung des Oxidationsmittels in der erfindungsgemäßen Oxidationszusammensetzung ist es bevorzugt, so genannte Komplexbildner einzusetzen. Komplexbildner sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt. Die Zahl der gebundenen Liganden hängt von der Koordinationszahl des zentralen Ions ab. Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di-oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Auch Dipicolinsäure wird erfindungsgemäß vorzugsweise als Komplexbildner eingesetzt. Mittel, die eine Kombination aus einem EDTA-Salz und HEDP und Dipicolinsäure enthalten sind erfindungsgemäß besonders bevorzugt. Weiterhin erfindungsgemäß bevorzugte Oxidationszusammensetzungen sind folgendermaßen zusammengesetzt, wobei alle Mengenangaben auf das Gewicht der Oxidationszusammensetzung bezogen sind:
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- In einer Gesamtmenge von 0,05 - 3 Gew.-% mindestens einen Polyethylenglycolether eines Monoesters von Glycerin und einer C12-C22-Carbonsäure mit 110 - 500 Ethylenoxideinheiten im Molekül gemäß der nachstehenden Formel (I) 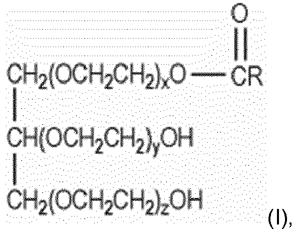 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
   wobei 0,05 - 3 Gew.-% PEG-200 Hydrogenated Glyceryl Palmate enthalten sind,
   wobei alle Mengenangaben auf das Gewicht der Oxidationszusammensetzung bezogen sind;
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- in einer Gesamtmenge von 0,05 - 3 Gew.-% mindestens einen Polyethylenglycolether eines Monoesters von Glycerin und einer C12-C22-Carbonsäure mit 110 - 500 Ethylenoxideinheiten im Molekül gemäß der nachstehenden Formel (I) 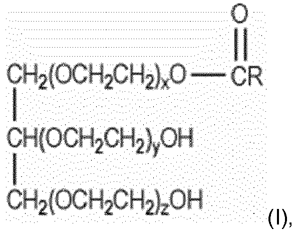 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
- in einer Gesamtmenge von 0,03 - 3 Gew.-% mindestens ein Polyethylenglycolether eines Monoesters von Glycerin und einer C8-C22-Carbonsäure mit 5 - 10 Ethylenoxideinheiten im Molekül gemäß der nachstehenden Formel (II), 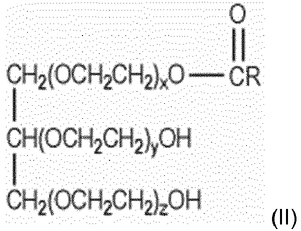 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann,
   wobei 0,05 - 3 Gew.-% PEG-200 Hydrogenated Glyceryl Palmate und 0,03 - 3 Gew.-% PEG-7 Glyceryl Cocoate enthalten sind,
   wobei alle Mengenangaben auf das Gewicht der Oxidationszusammensetzung bezogen sind;
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- in einer Gesamtmenge von 0,05 - 3 Gew.-% mindestens einen Polyethylenglycolether eines Monoesters von Glycerin und einer C12-C22-Carbonsäure mit 110 - 500 Ethylenoxideinheiten im Molekül gemäß der nachstehenden Formel (I) 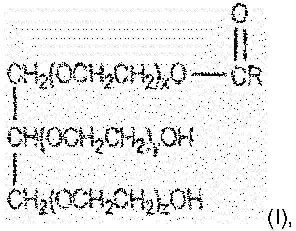 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
- in einer Gesamtmenge von 0,03 - 3 Gew.-% mindestens ein Polyethylenglycolether eines Monoesters von Glycerin und einer C8-C22-Carbonsäure mit 5 - 10 Ethylenoxideinheiten im Molekül gemäß der nachstehenden Formel (II), 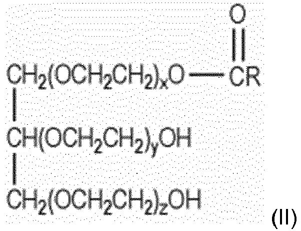 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann,
- mindestens ein lineares gesättigtes 1-Alkanol mit 12 - 30 Kohlenstoffatomen in einer Gesamtmenge von 2,7 - 6 Gew.-%, besonders bevorzugt 3,0 bis 5,0 Gew.-%, wobei 0,05 - 3 Gew.-% PEG-200 Hydrogenated Glyceryl Palmate und 0,03 - 3 Gew.-% PEG-7 Glyceryl Cocoate und 2,7 - 6 Gew.-% Cetearylalkohol enthalten sind,
   wobei alle Mengenangaben auf das Gewicht der Oxidationszusammensetzung bezogen sind;
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- in einer Gesamtmenge von 0,05 - 3 Gew.-% mindestens einen Polyethylenglycolether eines Monoesters von Glycerin und einer C12-C22-Carbonsäure mit 110 - 500 Ethylenoxideinheiten im Molekül gemäß der nachstehenden Formel (I) 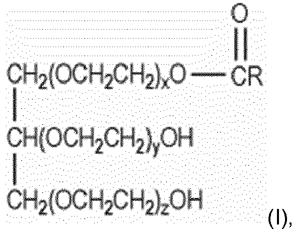 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
- in einer Gesamtmenge von 0,03 - 3 Gew.-% mindestens ein Polyethylenglycolether eines Monoesters von Glycerin und einer C8-C22-Carbonsäure mit 5 - 10 Ethylenoxideinheiten im Molekül gemäß der nachstehenden Formel (II), 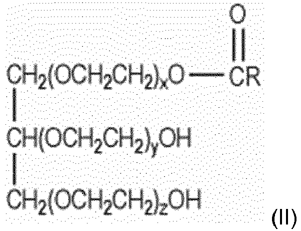 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann,
- mindestens ein lineares gesättigtes 1-Alkanol mit 12 - 30 Kohlenstoffatomen in einer Gesamtmenge von 2,7 - 6 Gew.-%, besonders bevorzugt 3,0 bis 5,0 Gew.-%,
- mindestens ein Polyethylenglycolether eines linearen gesättigten C12-C22-Alkanols mit 15 - 30 Ethylenoxideinheiten im Molekül in einer Gesamtmenge von 0,1 - 2 Gew.-%, besonders bevorzugt 0,5 bis 5,0 Gew.-%,
   wobei 0,05 - 3 Gew.-% PEG-200 Hydrogenated Glyceryl Palmate und 0,03 - 3 Gew.-% PEG-7 Glyceryl Cocoate und 2,7 - 6 Gew.-% Cetearylalkohol und 0,1 - 2 Gew.-% Ceteareth-20 enthalten sind,
   wobei alle Mengenangaben auf das Gewicht der Oxidationszusammensetzung bezogen sind;
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- in einer Gesamtmenge von 0,05 - 3 Gew.-% mindestens einen Polyethylenglycolether eines Monoesters von Glycerin und einer C12-C22-Carbonsäure mit 110 - 500 Ethylenoxideinheiten im Molekül gemäß der nachstehenden Formel (I) 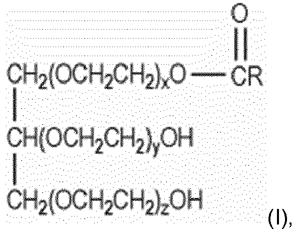 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
- in einer Gesamtmenge von 0,03 - 3 Gew.-% mindestens ein Polyethylenglycolether eines Monoesters von Glycerin und einer C8-C22-Carbonsäure mit 5 - 10 Ethylenoxideinheiten im Molekül gemäß der nachstehenden Formel (II), 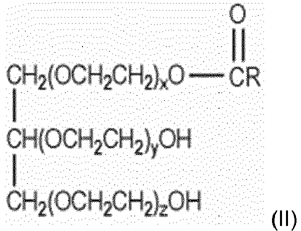 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann,
- mindestens ein lineares gesättigtes 1-Alkanol mit 12 - 30 Kohlenstoffatomen in einer Gesamtmenge von 2,7 - 6 Gew.-%, besonders bevorzugt 3,0 bis 5,0 Gew.-%,
- mindestens ein Polyethylenglycolether eines linearen gesättigten C12-C22-Alkanols mit 15 - 30 Ethylenoxideinheiten im Molekül in einer Gesamtmenge von 0,1 - 2 Gew.-%, besonders bevorzugt 0,5 bis 5,0 Gew.-%,
- mindestens ein Öl, das bevorzugt ausgewählt ist aus Mineralölen und Paraffinölen, in einer Gesamtmenge von 0,1 - 5 Gew.-%, besonders bevorzugt 0,2 - 3 Gew.-%, außerordentlich bevorzugt 0,3 - 1 Gew.-%,
   wobei 0,05 - 3 Gew.-% PEG-200 Hydrogenated Glyceryl Palmate und 0,03 - 3 Gew.-% PEG-7 Glyceryl Cocoate und 2,7 - 6 Gew.-% Cetearylalkohol und 0,1 - 2 Gew.-% Ceteareth-20 und 0,3 - 1 Gew.-% Paraffinöl enthalten sind,
   wobei alle Mengenangaben auf das Gewicht der Oxidationszusammensetzung bezogen sind.

Ein weiterer Gegenstand der vorliegenden Anmeldung ist ein Kit zur oxidativen Farbveränderung keratinischer Fasern, enthaltend zwei voneinander getrennte Zusammensetzungen (A) und (B), wobei die Zusammensetzung (B) eine erfindungsgemäße oder erfindungsgemäß bevorzugte Oxidationszusammensetzung, wie vorstehend beschrieben ist, also eine Oxidationszusammensetzung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens einen Polyethylenglycolether eines Monoesters von Glycerin und einer C12-C22-Carbonsäure mit 110 - 500 Ethylenoxideinheiten im Molekül gemäß der nachstehenden Formel (I) 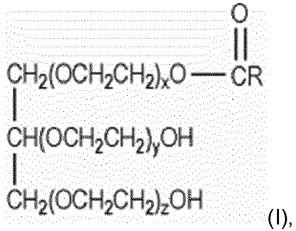 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
wobei alle Mengenangaben auf das Gewicht der Oxidationszusammensetzung bezogen sind;
und die Zusammensetzung (A) in Form einer Öl-in-Wasser-Emulsion vorliegt und mindestens ein Alkalisierungsmittel und gegebenenfalls mindestens ein Oxidationsfarbstoffvorprodukt enthält und einen pH-Wert im Bereich von 8 bis 11,5, gemessen bei 20°C, aufweist,
wobei die Zusammensetzungen (A) und (B) bevorzugt in einem gewichtsbezogenen Verhältnis (A)/(B) im Bereich von 0,33 - 3, besonders bevorzugt 0,5 - 2, außerordentlich bevorzugt 1:1, vorliegen.

Die Zusammensetzung (A) entspricht der vorstehend beschriebenen Alkalisierungszubereitung.

Die Zusammensetzung (A) enthält mindestens ein Alkalisierungsmittel, das ausgewählt ist aus der Gruppe, die gebildet wird von Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel sind ausgewählt aus Monoethanolamin, 2-Amino-2-methylpropanol und Triethanolamin. Die als erfindungsgemäßes Alkalisierungsmittel einsetzbaren basischen Aminosäuren sind bevorzugt ausgewählt aus der Gruppe, die gebildet wird aus Arginin, Lysin, Ornithin und Histidin, besonders bevorzugt Arginin.

Die Zusammensetzung (A) enthält mindestens ein Alkalisierungsmittel, das bevorzugt ausgewählt ist aus Ammoniak und Monoethanolamin sowie Mischungen dieser Alkalisierungsmittel.

Üblicherweise wird Ammoniak (NH₃) in Form seiner wässrigen Lösung eingesetzt. Wässrige Ammoniak-Lösungen enthalten Ammoniak (NH₃) oft in Konzentrationen von 10 bis 32 Gew.-%. Bevorzugt ist hierbei der Einsatz einer wässrigen Ammoniak-Lösung, die 25 Gew.-% Ammoniak (NH₃) enthält. Bevorzugt ist die Gesamtmenge an Alkalisierungsmitteln so gewählt, dass die Mischung, also das anwendungsbereite Farbveränderungsmittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist. Bevorzugt sind Ammoniak und/oder Monoethanolamin in der erfindungsgemäß verwendeten Zusammensetzung (A) in Mengen von 0,01 - 10 Gew.-%, bevorzugt von 0,1 bis 7,5 Gew.-%, weiter bevorzugt von 0,2 bis 5,5 Gew.-% und besonders bevorzugt von 0,4 bis 4,5 Gew.-% - jeweils bezogen auf das Gewicht der Zusammensetzung (A) - enthalten, so dass der pH-Wert der Zusammensetzung (A) im Bereich von 8 bis 11,5 liegt, gemessen bei 20°C.

Als optionalen Inhaltsstoff enthält die erfindungsgemäß verwendete emulsionsförmige Alkalisierungszusammensetzung (A) mindestens ein Oxidationsfarbstoffvorprodukt, das vorzugsweise aus einer oder mehreren Entwicklerkomponenten und gegebenenfalls einer oder mehreren Kupplerkomponenten ausgewählt ist.

Bevorzugt ist mindestens ein Oxidationsfarbstoffvorprodukt in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Bevorzugt ist mindestens eine Entwicklerkomponente in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Erfindungsgemäß bevorzugte Kupplerkomponenten sind ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Bevorzugt ist mindestens eine Kupplerkomponente in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Erfindungsgemäß bevorzugte Kits zur oxidativen Farbveränderung keratinischer Fasern sind dadurch gekennzeichnet, dass die vorgenannte erfindungsgemäße oder erfindungsgemäß bevorzugte Oxidationszusammensetzung (B) und die vorgenannte emulsionsförmige Alkalisierungszusammensetzung (A) in einem Gewichtsverhältnis A/B von 0,33 bis 3, besonders bevorzugt von 0,5 bis 2 und außerordentlich bevorzugt im Gewichtsverhältnis von 1:1, enthalten sind. Erfindungsgemäß besonders bevorzugte Kits zur oxidativen Farbveränderung keratinischer Fasern sind dadurch gekennzeichnet, dass die vorgenannte erfindungsgemäße oder erfindungsgemäß bevorzugte Oxidationszusammensetzung (B) und die vorgenannte emulsionsförmige Alkalisierungszusammensetzung (A) in einem Gewichtsverhältnis A/B von 0,33 bis 3, besonders bevorzugt von 0,5 bis 2 und außerordentlich bevorzugt im Gewichtsverhältnis von 1 :1, enthalten sind, wobei das Kit keine weiteren Komponenten enthält, die zur anwendungsbereiten Farbveränderungsmischung zugegeben werden, während Komponenten zur Vor- oder Nachbehandlung der keratinischen Fasern, beispielsweise Conditioner oder Shampoos, im Kit enthalten sein können.

In einer erfindungsgemäß bevorzugten Ausführungsform ist das erfindungsgemäße Kit dadurch gekennzeichnet, dass die Zusammensetzung (A), jeweils bezogen auf ihr Gewicht, 50 - 80 Gew.-% Wasser, weiterhin 5 - 20 Gew.-%, bevorzugt 8 - 15 Gew.-% an mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern aus ein- und mehrwertigen C1-C10-Alkanolen und C8-C30-Alkansäuren sowie Mischungen hiervon, weiterhin mindestens ein nichtionisches und/oder anionisches Tensid in einer Gesamtmenge von 1 - 8 Gew.-%, bevorzugt 2 - 6 Gew.-%, enthält.

Geeignete Fettkomponenten mit einem Schmelzpunkt im Bereich von 28 - 80 °C sind beispielsweise ausgewählt aus Estern aus einem gesättigten, einwertigen C₁₆-C₆₀-Alkanol und einer gesättigten Cs-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren, weiterhin Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren, z. B. Stearyllaurat, Cetearylstearat, Cetylpalmitat und Myristylmyristat, weiterhin die vorgenannten linearen gesättigten 1-Alkanole mit 12 - 30 Kohlenstoffatomen, insbesondere Laurylalkohol, Myristylalkohol, Cetylalkohol, Stearylalkohol, Arachidylalkohol, und Behenylalkohol, weiterhin Glycerylmonostearat, Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, sowie Mischungen der vorgenannten Substanzen.

Geeignete nichtionische Tenside sind beispielsweise Alkylenoxid-Anlagerungsprodukte an Fettalkohole und Fettsäuren mit jeweils 2 bis 50 Mol Ethylenoxid pro Mol Fettalkohol bzw. Fettsäure, sowie weiterhin Alkyl- oder Alkenyloligoglykoside, z. B. Decylglucosid.

Geeignete anionische Tenside sind beispielsweise Fettsäuren, Alkylsulfate, Alkylethersulfate und Ethercarbonsäuren mit 10 bis 20 C-Atomen in der Alkylgruppe und bis zu 16 Glykolethergruppen im Molekül.

Bezüglich weiterer bevorzugter Ausführungsformen des erfindungsgemäßen Kits gilt mutatis mutandis das zu den erfindungsgemäßen und erfindungsgemäß bevorzugten Oxidationszusammensetzungen sowie das zu den erfindungsgemäß verwendeten emulsionsförmigen Alkalisierungszusammensetzungen (A) Gesagte.

Ein weiterer Gegenstand der vorliegenden Anmeldung ist ein Verfahren zur oxidativen Farbveränderung keratinischer Fasern, das durch folgende Verfahrensschritte gekennzeichnet ist:
Bereitstellung einer Oxidationszusammensetzung (B) gemäß einem der Ansprüche 1 - 11, enthaltend
   - 50 - 96 Gew.-%, bevorzugt 70 - 93 Gew.-%, besonders bevorzugt 80 - 90 Gew.-%, Wasser,
   - 0,5 - 20 Gew.-% Wasserstoffperoxid,
   - mindestens einen Polyethylenglycolether eines Monoesters von Glycerin und einer C12-C22-Carbonsäure mit 110 - 500 Ethylenoxideinheiten im Molekül gemäß der nachstehenden Formel (I) 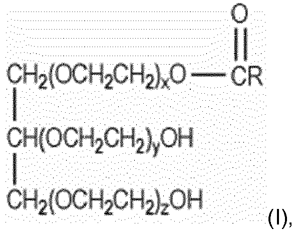 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
   wobei alle Mengenangaben auf das Gewicht der Oxidationszusammensetzung bezogen sind, und
Bereitstellung einer Zusammensetzung (A), die in Form einer Öl-in-Wasser-Emulsion vorliegt und mindestens ein Alkalisierungsmittel und gegebenenfalls mindestens ein Oxidationsfarbstoffvorprodukt enthält und einen pH-Wert im Bereich von 8 bis 11,5, gemessen bei 20°C, aufweist und weiterhin bevorzugt, jeweils bezogen auf ihr Gewicht, 50 - 80 Gew.-% Wasser, weiterhin bevorzugt 5 - 20 Gew.-%, besonders bevorzugt 8 - 15 Gew.-% an mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern aus ein- und mehrwertigen C1-C10-Alkanolen und C8-C30-Alkansäuren sowie Mischungen hiervon, weiterhin bevorzugt mindestens ein nichtionisches und/oder anionisches Tensid in einer Gesamtmenge von 1 - 8 Gew.-%, bevorzugt 2 - 6 Gew.-%, enthält,
Herstellung einer Mischung aus der vorgenannten Oxidationszusammensetzung (B) und der vorgenannten Zusammensetzung (A), bevorzugt im gewichtsbezogenen Mischungsverhältnis (A)/(B) im Bereich von 0,33 - 3, besonders bevorzugt 0,5 - 2, außerordentlich bevorzugt 1:1, unmittelbar danach Verteilung des anwendungsbereiten Mittels auf den Fasern,
Verbleib des Mittels auf den Fasern für einen Zeitraum von 1 bis 60 Minuten, unmittelbar danach
Ausspülen des verbliebenen Mittels aus den Fasern und ggf. Trocknen der Fasern.

Erfindungsgemäß bevorzugte Verfahren zur oxidativen Farbveränderung keratinischer Fasern sind dadurch gekennzeichnet, dass die vorgenannte Oxidationszusammensetzung (B) und die vorgenannte emulsionsförmige Alkalisierungszusammensetzung (A) im gewichtsbezogenen Mischungsverhältnis (A)/(B) im Bereich von 0,33 - 3, besonders bevorzugt 0,5 - 2, außerordentlich bevorzugt 1:1, miteinander vermischt werden.

Weitere erfindungsgemäße Ausführungsformen der Oxidationszusammensetzung, des Kits und des Färbeverfahrens sind dadurch gekennzeichnet, dass sowohl die Oxidationszusammensetzung (B) als auch die Zusammensetzung (A) kationische Tenside, Polysaccharide und (Co-)Polymere aus (Meth)acrylsäure, (Meth)acrylsäureestern, (Meth)acrylsäureamiden oder quaternisierten (Meth)acrylsäure-basierten Monomeren in einer Gesamtmenge von 0 bis 0,5 Gew.-%, bevorzugt 0,05 bis 0,3 Gew.-%, besonders bevorzugt 0,08 bis 0,2 Gew.-%, jeweils bezogen auf das Gewicht der Einzelzusammensetzung (A) oder (B), enthalten.

Es hat sich in bestimmten Fällen als ungünstig für die Anwendungseigenschaften, als auch für die Hautverträglichkeitseigenschaften des anwendungsbereiten Farbveränderungsmittels erwiesen, wenn die Zusammensetzungen (A) und/oder (B) mehr als 0,5 Gew.-% an kationischen Tensiden, Polysacchariden und (Co-)Polymeren aus (Meth)acrylsäure, (Meth)acrylsäureestern, (Meth)acrylsäureamiden oder quaternisierten (Meth)acrylsäure-basierten Monomeren enthalten.

Bezüglich weiterer bevorzugter Ausführungsformen des erfindungsgemäßen Verfahrens gilt mutatis mutandis das zu den erfindungsgemäßen und erfindungsgemäß bevorzugten Oxidationszusammensetzungen sowie das zu den erfindungsgemäß verwendeten emulsionsförmigen Alkalisierungszusammensetzungen (A) Gesagte.

Unter keratinischen bzw. keratinhaltigen Fasern sind erfindungsgemäß Wolle, Pelze, Federn und insbesondere menschliche Haare zu verstehen. Die erfindungsgemäßen Färbe- und/oder Aufhellverfahren können prinzipiell aber auch zur Anwendung auf anderen Naturfasern, wie z. B. Baumwolle, Jute, Sisal, Leinen, Seide oder modifizierten Naturfasern, wie beispielsweise Regeneratcellulose, Nitro-, Alkyl- oder Hydroxyalkyl- oder Acetylcellulose, verwendet werden.

Das anwendungsbereite Färbemittel des erfindungsgemäßen Verfahrens wird bevorzugt durch Zusammenführen der erfindungsgemäßen oder erfindungsgemäß bevorzugten Oxidationszusammensetzung mit einer erfindungsgemäß verwendeten emulsionsförmige Alkalisierungszusammensetzung (A) in einem wiederverschließbaren Behältnis und anschließendes Vermischen hergestellt. Im anschließenden Verfahrensschritt wird das anwendungsbereite Färbemittel auf den keratinischen Fasern verteilt. Bei dem Verfahren zur Farbveränderung menschlicher Haare wird das anwendungsbereite Mittel dabei direkt auf dem Kopfhaar des Anwenders verteilt. Bevorzugt erfolgt die Verteilung manuell. Dazu entnimmt der Anwender das anwendungsbereite Mittel dem Mischungsbehältnis, bevorzugt dem wiederverschließbaren Behältnis, durch Schöpfen oder Gießen auf die Hand und anschließende Verteilung und bevorzugt Einarbeitung des Mittels auf dem Kopfhaar. Bevorzugt wird dabei der direkte Kontakt zwischen anwendungsbereitem Farbveränderungsmittel und den Händen durch die Verwendung von geeigneten Handschuhen, wie Einweghandschuhen, beispielsweise aus Latex, vermieden.

Anschließend verbleibt das anwendungsbereite Färbemittel für einen Zeitraum von 1 bis 60 min auf den zu behandelnden Fasern. Bevorzugt liegt der Zeitraum im Bereich von 10 bis 45 min, besonders bevorzugt 20 bis 30 Minuten.

Die Anwendungstemperaturen können in einem Bereich zwischen 15 und 40 °C liegen. Gegebenenfalls kann während der Verbleibzeit des Mittels auf den Fasern auch durch externe Wärmequellen eine höhere oder genau definierte Temperatur eingestellt werden. Besonders bevorzugt ist es, die Farbveränderung durch physikalische Maßnahmen zu unterstützen. Erfindungsgemäße Verfahren, bei denen die Anwendung durch Einwirkung von Wärme, IR- und/oder UV-Strahlung während der Einwirkzeit unterstützt wird, können dabei bevorzugt sein.

Nach Ablauf der Einwirkungszeit wird das anwendungsbereite Färbemittel bzw. das verbleibende Färbemittel im letzten Verfahrensschritt durch Ausspülen von den zu behandelnden Fasern entfernt. Hierzu werden die Fasern mit Wasser und/oder einer wässrigen Tensid-Zubereitung ausgespült. Üblicherweise wird hierzu 20 °C bis 40 °C warmes Wasser bzw. eine entsprechend temperierte wässrige Tensid-Zubereitung eingesetzt. Optional können sich hieran weitere Behandlungsschritte anschließen, wie das Auftragen eines Leave-on oder Rinse-off-Conditioners, ein weiterer Färbeschritt, z.B. das Einfärben oder Aufhellen von Strähnchen, eine Haarverformung und/oder das Trocknen der Haare.

### Beispiele

Es wurden die folgenden Zubereitungen hergestellt. Tabelle 1 enthält ein Beispiel für eine O/Wemulsionsförmige Alkalisierungszusammensetzung (A); Tabelle 2 enthält ein Beispiel für eine erfindungsgemäße Oxidationszusammensetzung (B). Die Mengenangaben sind - sofern nicht anders angegeben - in Gew.-%, jeweils bezogen das Gewicht der einzelnen Zusammensetzung.

**Tabelle 1: emulsionsförmige (O/W) Alkalisierungszusammensetzung (A) (Färbezubereitung)**

| | |
|---|---|
| Cetearylalkohol | 13,0 |
| Ammoniumhydroxid | 6,4 |
| Glycerylstearat | 3,5 |
| Monoethanolamin | 1,0 |
| Ceteareth-20 | 3,5 |
| Octyldodecanol | 2,0 |
| Natriumlaurethsulfat | 0,9 |
| Natriumcetearylsulfat | 0,5 |
| Serin | 0,5 |
| Parfum | 0,5 |
| Ölsäure | 0,3 |
| Silica | 0,25 |
| Kaliumstearat | 0,5 |
| Titandioxid | 0,7 |
| Glycerin | 1,5 |
| Tetrasodium EDTA | 0,2 |
| Toluene-2,5-Diaminsulfat | 0,15 |
| Natriumsulfit | 0,15 |
| Carbomer | 0,2 |
| Keratinhydrolysat | 0,1 |
| Kaliumhydroxid | 0,06 |
| Resorcin | 0,06 |
| Ascorbinsäure | 0,05 |
| Linoleamidopropyl PG-Dimonium Chloride Phosphate | 0,1 |
| Propylenglycol | 1,0 |
| 2-Amino-4-Hydroxyethylaminoanisole Sulfate | 0,01 |
| m-Aminophenol | 0,01 |
| Aqua | ad 100 |

**Tabelle 2: erfindungsgemäße Oxidationszusammensetzungen (B)**

| | | |
|---|---|---|
| 2,6-Dicarboxypyridine | 0,1 | 0,1 |
| Sodium Benzoate | 0,04 | 0,04 |
| Disodium Pyrophosphate | 0,1 | 0,1 |
| Etidronic Acid | 0,15 | 0,15 |
| Potassium Hydroxide | 0,095 | 0,095 |
| Cetearyl Alcohol | 3,4 | 3,4 |
| Ceteareth-20 | 1 | 1 |
| Paraffinum Liquidum (Mineral Oil) | 0,3 | 0,3 |
| PEG-7 Glyceryl Cocoate | 0,1 | 1,0 |
| PEG-200 Hydrogenated Glyceryl Palmate | 0,4 | 0,2 |
| Hydrogen Peroxide | 6,0 | 6,0 |
| Aqua (Water, Eau) | ad 100 | ad 100 |

### Ausfärbung

100 g der Färbezubereitung wurden mit 100 g der erfindungsgemäßen Oxidationszusammensetzung in einem wiederverschließbaren Mischungsbehältnis durch kräftiges, ununterbrochenes Schütteln (40mal) miteinander vermischt.

Die Produkte wurden jeweils aus den Behältnissen mit der hohlen Hand entnommen und gleichmäßig auf zwei identische Haarsträhnen (Alkino) verteilt.

Das jeweilige anwendungsbereite Mittel verblieb für eine Einwirkdauer von 30 min bei Raumtemperatur (20°C) auf den Haarsträhnen, ohne herunterzutropfen.

Anschließend wurden die verbliebenen Mittel jeweils mit lauwarmen Wasser (25°C) aus den Haarsträhnen ca. 2 min lang ausgespült, und die Strähnen wurden mit einem Handtuch getrocknet.

Es wurden gleichmäßige, haltbare und glänzende Färbungen von hoher Farbintensität und Farbigkeit erhalten.

## Patentansprüche

1. Oxidationszusammensetzung zur oxidativen Haarbehandlung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens einen Polyethylenglycolether eines Monoesters von Glycerin und einer C12-C22-Carbonsäure mit 110 - 500 Ethylenoxideinheiten im Molekül gemäß der nachstehenden Formel (I) 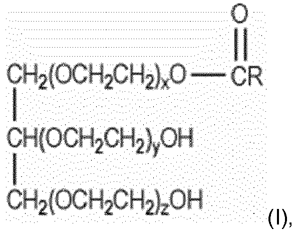 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
wobei alle Mengenangaben auf das Gewicht der Oxidationszusammensetzung bezogen sind.

2. Zusammensetzung gemäß Anspruch 1, **dadurch gekennzeichnet, dass** der Polyethylenglycolether gemäß Formel (I) ausgewählt ist aus Verbindungen, bei denen die Summe der Indizes x + y + z für eine Zahl im Bereich von 120 bis 450, bevorzugt für eine Zahl im Bereich von 150 bis 300, besonders bevorzugt für eine Zahl im Bereich von 200 bis 250, steht.

3. Zusammensetzung gemäß Anspruch 1 oder 2, **dadurch gekennzeichnet, dass** der Polyethylenglycolether gemäß Formel (I) ausgewählt ist aus Verbindungen, bei denen R für einen linearen Alkylrest mit 15 bis 17 C-Atomen steht, der bevorzugt gesättigt ist, steht, wobei der Polyethylenglycolether gemäß Formel (I) bevorzugt ausgewählt ist aus PEG-200 Glycerylmonopalmitat, PEG-200 Glycerylmonostearat, PEG-120 Glycerylmonopalmitat, PEG-120 Glycerylmonostearat, PEG-150 Glycerylmonopalmitat, PEG-150 Glycerylmonostearat, PEG-300 Glycerylmonopalmitat, PEG-300 Glycerylmonostearat, PEG-200 Glycerylmonopalmat, PEG-200 Hydrogenated Glyceryl Palmate sowie Mischungen hiervon, besonders bevorzugt ausgewählt ist aus PEG-200 Hydrogenated Glyceryl Palmate.

4. Zusammensetzung gemäß einem der Ansprüche 1 bis 3, **dadurch gekennzeichnet, dass** mindestens ein Polyethylenglycolether gemäß Formel (I) in einer Gesamtmenge von 0,05 bis 3 Gew.-%, bevorzugt 0,1 bis 2 Gew.-%, besonders bevorzugt 0,2 - 0,7 Gew.-%, jeweils bezogen auf das Gewicht der Oxidationszusammensetzung, enthalten ist.

5. Zusammensetzung gemäß einem der Ansprüche 1 bis 4, **dadurch gekennzeichnet, dass** mindestens ein Polyethylenglycolether eines Monoesters von Glycerin und einer C8-C22-Carbonsäure mit 5 - 10 Ethylenoxideinheiten im Molekül gemäß der nachstehenden Formel (II), 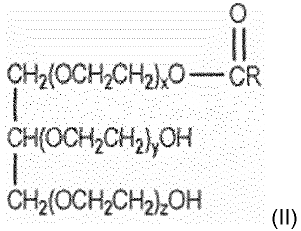 wobei die Summe Indizes x + y + z für eine Zahl im Bereich von 5 bis 10 steht und R für einen Alkylrest mit 7 bis 21 C-Atomen steht, der gesättigt oder ungesättigt, linear oder verzweigt sein kann,
enthalten ist.

6. Zusammensetzung gemäß einem der Ansprüche 1 bis 5, **dadurch gekennzeichnet, dass** der Polyethylenglycolether gemäß Formel (II) ausgewählt ist aus Verbindungen, bei denen die Summe der Indizes x + y + z für eine Zahl im Bereich von 5 bis 10, bevorzugt für eine Zahl im Bereich von 7 - 9, und R für einen linearen Alkylrest mit 7 bis 17 C-Atomen steht, der bevorzugt gesättigt ist, wobei der Polyethylenglycolether gemäß Formel (II) bevorzugt ausgewählt ist aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, PEG-7 Glycerylcocoat sowie Mischungen hiervon, besonders bevorzugt ausgewählt aus PEG-7 Glycerylcocoat.

7. Zusammensetzung gemäß einem der Ansprüche 1 bis 6, **dadurch gekennzeichnet, dass** mindestens ein Polyethylenglycolether gemäß Formel (II) in einer Gesamtmenge von 0,03 bis 3 Gew.-%, bevorzugt 0,05 bis 2 Gew.-%, besonders bevorzugt 0,2 - 1,5 Gew.-%, außerordentlich bevorzugt 0,5 bis 1,0 Gew.-%, jeweils bezogen auf das Gewicht der Oxidationszusammensetzung, enthalten ist.

8. Zusammensetzung gemäß einem der Ansprüche 1 bis 7, **dadurch gekennzeichnet, dass** mindestens ein lineares gesättigtes 1-Alkanol mit 12 - 30 Kohlenstoffatomen enthalten ist, bevorzugt in einer Gesamtmenge von 2,7 - 6 Gew.-%, besonders bevorzugt 3,0 bis 5,0 Gew.-%, jeweils bezogen auf das Gewicht der Oxidationszusammensetzung.

9. Zusammensetzung gemäß einem der Ansprüche 1 bis 8, **dadurch gekennzeichnet, dass** mindestens ein Polyethylenglycolether eines linearen gesättigten C12-C22-Alkanols mit 15 - 30 Ethylenoxideinheiten im Molekül enthalten ist, bevorzugt in einer Gesamtmenge von 0,1 - 2 Gew.-%, besonders bevorzugt 0,5 bis 5,0 Gew.-%, jeweils bezogen auf das Gewicht der Oxidationszusammensetzung.

10. Zusammensetzung gemäß einem der Ansprüche 1 bis 9, **dadurch gekennzeichnet, dass** mindestens ein Öl, das bevorzugt ausgewählt ist aus Mineralölen und Paraffinölen, enthalten ist, bevorzugt in einer Gesamtmenge von 0,1 - 5 Gew.-%, besonders bevorzugt 0,2 - 3 Gew.-%, außerordentlich bevorzugt 0,3 - 1 Gew.-%, jeweils bezogen auf das Gewicht der Oxidationszusammensetzung.

11. Zusammensetzung nach einem der Ansprüche 1 bis 8, **dadurch gekennzeichnet, dass** sie einen pH-Wert im Bereich von 2,5 bis 5,5 und eine Viskosität im Bereich von 1500 bis 3500 mPas, bevorzugt 2000 bis 3000 mPas, gemessen bei 20°C, aufweist.

12. Kit zur oxidativen Farbveränderung keratinischer Fasern, enthaltend zwei voneinander getrennte Zusammensetzungen (A) und (B), wobei
die Zusammensetzung (B) eine Oxidationszusammensetzung nach einem der Ansprüche 1 - 11 ist und
die Zusammensetzung (A) in Form einer Öl-in-Wasser-Emulsion vorliegt und mindestens ein Alkalisierungsmittel und gegebenenfalls mindestens ein Oxidationsfarbstoffvorprodukt enthält und einen pH-Wert im Bereich von 8 bis 11,5, gemessen bei 20°C, aufweist,
wobei die Zusammensetzungen (A) und (B) bevorzugt in einem gewichtsbezogenen Verhältnis (A)/(B) im Bereich von 0,33 - 3, besonders bevorzugt 0,5 - 2, außerordentlich bevorzugt 1:1, vorliegen.

13. Kit zur oxidativen Farbveränderung keratinischer Fasern nach Anspruch 12, **dadurch gekennzeichnet, dass** die Zusammensetzung (A), jeweils bezogen auf ihr Gewicht, 50 - 80 Gew.-% Wasser, weiterhin 5 - 20 Gew.-%, bevorzugt 8 - 15 Gew.-% an mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern aus ein- und mehrwertigen C1-C10-Alkanolen und C8-C30-Alkansäuren sowie Mischungen hiervon, weiterhin mindestens ein nichtionisches und/oder anionisches Tensid in einer Gesamtmenge von 1 - 8 Gew.-%, bevorzugt 2 - 6 Gew.-%, enthält.

14. Verfahren zur oxidativen Farbveränderung keratinischer Fasern, **gekennzeichnet durch** folgende Verfahrensschritte:
Bereitstellung einer Oxidationszusammensetzung (B) gemäß einem der Ansprüche 1 - 11, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens einen Polyethylenglycolether eines Monoesters von Glycerin und einer C12-C22-Carbonsäure mit 110 - 500 Ethylenoxideinheiten im Molekül gemäß der nachstehenden Formel (I) 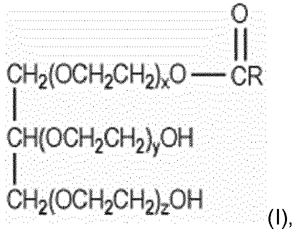 wobei die Summe der Indizes x + y + z für eine Zahl im Bereich von 110 bis 500 steht und R für einen Alkylrest mit 11 bis 21 C-Atomen steht, der gesättigt oder ungesättigt, linear oder verzweigt sein kann,
wobei alle Mengenangaben auf das Gewicht der Oxidationszusammensetzung bezogen sind, und
Bereitstellung einer Zusammensetzung (A), die in Form einer Öl-in-Wasser-Emulsion vorliegt und mindestens ein Alkalisierungsmittel und gegebenenfalls mindestens ein Oxidationsfarbstoffvorprodukt enthält und einen pH-Wert im Bereich von 8 bis 11,5, gemessen bei 20°C, aufweist und weiterhin bevorzugt, jeweils bezogen auf ihr Gewicht, 50 - 80 Gew.-% Wasser, weiterhin bevorzugt 5 - 20 Gew.-%, besonders bevorzugt 8- 15 Gew.-% an mindestens einer Fettkomponente mit einem Schmelzpunkt im Bereich von 28 - 80 °C, die bevorzugt ausgewählt ist aus linearen gesättigten 1-Alkanolen mit 12 - 30 Kohlenstoffatomen und Estern aus ein- und mehrwertigen C1-C10-Alkanolen und C8-C30-Alkansäuren sowie Mischungen hiervon, weiterhin bevorzugt mindestens ein nichtionisches und/oder anionisches Tensid in einer Gesamtmenge von 1 - 8 Gew.-%, bevorzugt 2 - 6 Gew.-%, enthält,
Herstellung einer Mischung aus der vorgenannten Oxidationszusammensetzung (B) und der vorgenannten Zusammensetzung (A), bevorzugt im gewichtsbezogenen Mischungsverhältnis (A)/(B) im Bereich von 0,33 - 3, besonders bevorzugt 0,5 - 2, außerordentlich bevorzugt 1:1, unmittelbar danach Verteilung des anwendungsbereiten Mittels auf den Fasern,
Verbleib des Mittels auf den Fasern für einen Zeitraum von 1 bis 60 Minuten, unmittelbar danach
Ausspülen des verbliebenen Mittels aus den Fasern und ggf. Trocknen der Fasern.

15. Verfahren zur oxidativen Farbveränderung keratinischer Fasern nach Anspruch 14, **dadurch gekennzeichnet, dass** sowohl die Oxidationszusammensetzung (B) als auch die Zusammensetzung (A) kationische Tenside, Polysaccharide und (Co-)Polymere aus (Meth)acrylsäure, (Meth)acrylsäureestern, (Meth)acrylsäureamiden oder quaternisierten (Meth)acrylsäurebasierten Monomeren in einer Gesamtmenge von 0 bis 0,5 Gew.-%, bevorzugt 0,05 bis 0,3 Gew.-%, besonders bevorzugt 0,08 bis 0,2 Gew.-%, jeweils bezogen auf das Gewicht der Einzelzusammensetzung (A) oder (B), enthalten.

## Claims

1. An oxidation composition for oxidative hair treatment, containing
- 50-96 wt.%, preferably 70-93 wt.%, particularly preferably 80-90 wt.%, water,
- 0.5-20 wt.% hydrogen peroxide,
- at least one polyethylene glycol ether of a monoester of glycerol and a C12-C22 carboxylic acid having 110-500 ethylene oxide units in the molecule according to the following formula (I) 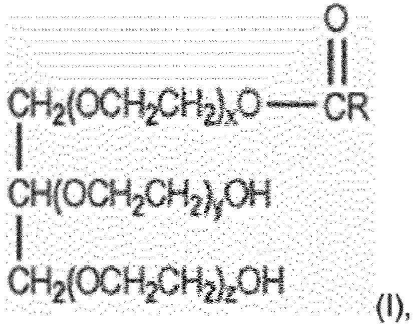 where the sum of the indices x + y + z represents a number in the range of from 110 to 500 and R represents an alkyl functional group which has 11 to 21 C atoms and can be saturated or unsaturated, linear or branched, where all the amounts stated are based on the weight of the oxidation composition.

2. The composition according to claim 1, **characterized in that** the polyethylene glycol ether according to formula (I) is selected from compounds in which the sum of the indices x + y + z represents a number in the range of from 120 to 450, preferably a number in the range of from 150 to 300, particularly preferably a number in the range of from 200 to 250.

3. The composition according to claim 1 or 2, **characterized in that** the polyethylene glycol ether according to formula (I) is selected from compounds in which R represents a linear alkyl functional group which has 15 to 17 C atoms and is preferably saturated, the polyethylene glycol ether according to formula (I) preferably being selected from PEG-200 glyceryl monopalmitate, PEG-200 glyceryl monostearate, PEG-120 glyceryl monopalmitate, PEG-120 glyceryl monostearate, PEG-150 glyceryl monopalmitate, PEG-150 glyceryl monostearate, PEG-300 glyceryl monopalmitate, PEG-300 glyceryl monostearate, PEG-200 glyceryl monopalmitate, PEG-200 hydrogenated glyceryl palmate, and mixtures thereof, particularly preferably selected from PEG-200 hydrogenated glyceryl palmate.

4. The composition according to one of claims 1 to 3, **characterized in that** at least one polyethylene glycol ether according to formula (I) is contained in a total amount of from 0.05 to 3 wt.%, preferably 0.1 to 2 wt.%, particularly preferably 0.2 to 0.7 wt.%, in each case based on the weight of the oxidation composition.

5. The composition according to one of claims 1 to 4, **characterized in that** at least one polyethylene glycol ether of a monoester of glycerol and a C8-C22 carboxylic acid having 5-10 ethylene oxide units in the molecule according to the following formula (II) is contained, 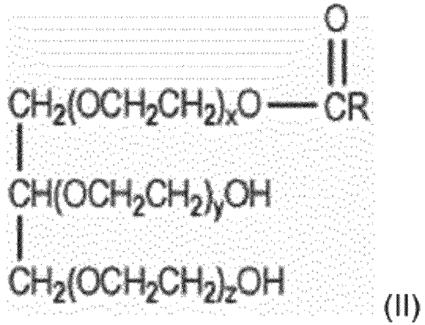 where the sum of the indices x + y + z represents a number in the range of from 5 to 10 and R represents an alkyl functional group which has 7 to 21 C atoms and can be saturated or unsaturated, linear or branched.

6. The composition according to one of claims 1 to 5, **characterized in that** the polyethylene glycol ether according to formula (II) is selected from compounds in which the sum of the indices x + y + z represents a number in the range of from 5 to 10, preferably a number in the range of from 7-9, and R represents a linear alkyl functional group which has 7 to 17 C atoms and is preferably saturated, the polyethylene glycol ether according to formula (II) preferably being selected from PEG-7 glyceryl monolaurate, PEG-7 glyceryl monomyristate, PEG-7 glyceryl monopalmitate, PEG-7 glyceryl monostearate, PEG-7 glyceryl monocaprylate, PEG-7 glyceryl monocaprate, PEG-10 glyceryl monolaurate, PEG-10 glyceryl monomyristate, PEG-10 glyceryl monopalmitate, PEG-10 glyceryl monostearate, PEG-10 glyceryl monocaprylate, PEG-10 glyceryl monocaprate, PEG-5 glyceryl monolaurate, PEG-5 glyceryl monomyristate, PEG-5 glyceryl monopalmitate, PEG-5 glyceryl monostearate, PEG-5 glyceryl monocaprylate, PEG-5 glyceryl monocaprate, PEG-7 glyceryl cocoate, and mixtures thereof, particularly preferably selected from PEG-7 hydrogenated glyceryl cocoate.

7. The composition according to one of claims 1 to 6, **characterized in that** at least one polyethylene glycol ether according to formula (II) is contained in a total amount of from 0.03 to 3 wt.%, preferably 0.05 to 2 wt.%, particularly preferably 0.2-1.5 wt.%, extremely preferably 0.5 to 1.0 wt.%, in each case based on the weight of the oxidation composition.

8. The composition according to one of claims 1 to 7, **characterized in that** at least one linear saturated 1-alkanol having 12-30 carbon atoms is contained, preferably in a total amount of 2.7-6 wt.%, particularly preferably 3.0 to 5.0 wt.%, in each case based on the weight of the oxidation composition.

9. The composition according to one of claims 1 to 8, **characterized in that** at least one polyethylene glycol ether of a linear saturated C12-C22 alkanol having 15-30 ethylene oxide units in the molecule is contained, preferably in a total amount of 0.1-2 wt.%, particularly preferably 0.5 to 5.0 wt.%, in each case based on the weight of the oxidation composition.

10. The composition according to one of claims 1 to 9, **characterized in that** at least one oil is contained, preferably selected from mineral oils and paraffin oils, preferably in a total amount of 0.1-5 wt.%, particularly preferably 0.2-3 wt.%, extremely preferably 0.3-1 wt.%, in each case based on the weight of the oxidation composition.

11. The composition according to one of claims 1 to 8, **characterized in that** it has a pH in the range of from 2.5 to 5.5 and a viscosity in the range of from 1500 to 3500 mPas, preferably 2000 to 3000 mPas, measured at 20 °C.

12. A kit for oxidatively changing the color of keratin fibers, containing two mutually separated compositions (A) and (B), wherein composition (B) is an oxidation composition according to one of claims 1-11 and composition (A) is in the form of an oil-in-water emulsion and contains at least one alkalizing agent and optionally at least one oxidation dye precursor and has a pH in the range of from 8 to 11.5, measured at 20 °C, wherein compositions (A) and (B) are preferably in a weight-based ratio (A)/(B) in the range of from 0.33-3, particularly preferably 0.5-2, extremely preferably 1:1.

13. The kit for oxidatively changing the color of keratin fibers according to claim 12, **characterized in that** composition (A) contains, in each case based on the weight thereof, 50-80 wt.% water, and 5-20 wt.%, preferably 8-15 wt.%, of at least one fat component having a melting point in the range of from 28-80 °C, preferably selected from linear saturated 1-alkanols having 12-30 carbon atoms and esters of mono- and polyvalent C1-C10 alkanols and C8-C30 alkanoic acids, and mixtures thereof, and at least one non-ionic and/or anionic surfactant in a total amount of 1-8 wt.%, preferably 2-6 wt.%.

14. A method for oxidatively changing the color of keratin fibers, **characterized by** the following method steps:
providing an oxidation composition (B) according to one of claims 1-11, containing
- 50-96 wt.%, preferably 70-93 wt.%, particularly preferably 80-90 wt.%, water,
- 0.5-20 wt.% hydrogen peroxide,
- at least one polyethylene glycol ether of a monoester of glycerol and a C12-C22 carboxylic acid having 110-500 ethylene oxide units in the molecule according to the following formula (I) 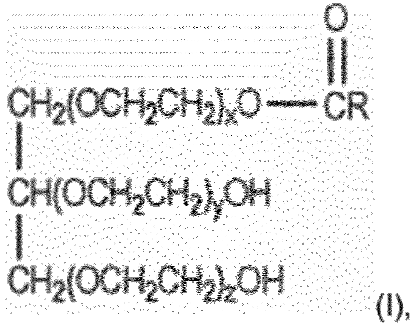
where the sum of the indices x + y + z represents a number in the range of from 110 to 500 and R represents an alkyl functional group which has 11 to 21 C atoms and can be saturated or unsaturated, linear or branched, where all the amounts stated are based on the weight of the oxidation composition, and providing a composition (A) which is in the form of an oil-in-water emulsion and contains at least one alkalizing agent and optionally at least one oxidation dye precursor and has a pH in the range of from 8 to 11.5, measured at 20 °C, and further preferably contains, in each case based on the weight thereof, 50-80 wt.% water, further preferably 5-20 wt.%, particularly preferably 8-15 wt.%, of at least one fat component having a melting point in the range of from 28-80 °C and preferably selected from linear saturated 1-alkanols having 12-30 carbon atoms and esters of mono- and polyvalent C1-C10 alkanols and C8-C30 alkanoic acids and mixtures thereof, further preferably at least one non-ionic and/or anionic surfactant in a total amount of 1-8 wt.%, preferably 2-6 wt.%, preparing a mixture of the above-mentioned oxidation composition (B) and the above-mentioned composition (A), preferably in a weight-based mixing ratio (A)/(B) in the range of from 0.33-3, particularly preferably 0.5-2, extremely preferably 1:1, and, directly thereafter, distributing the ready-to-use agent on the fibers, leaving the agent on the fibers for a period of from 1 to 60 minutes and, directly thereafter, rinsing out the remaining agent from the fibers and optionally drying the fibers.

15. The method for oxidatively changing the color of keratin fibers according to claim 14, **characterized in that** both oxidation composition (B) and composition (A) contain cationic surfactants, polysaccharides and (co-)polymers of (meth)acrylic acid, (meth)acrylic acid esters, (meth)acrylic acid amides or quaternized (meth)acrylic-acid-based monomers, in a total amount of from 0 to 0.5 wt.%, preferably 0.05 to 0.3 wt.%, particularly preferably 0.08 to 0.2 wt.%, in each case based on the weight of the individual composition (A) or (B).

## Revendications

1. Composition d'oxydation pour le traitement capillaire oxydatif, contenant :
- 50 à 96 % en poids, de préférence 70 à 93 % en poids, de manière particulièrement préférée 80 à 90 % en poids d'eau,
- 0,5 à 20 % en poids de peroxyde d'hydrogène,
- au moins un éther de polyéthylèneglycol d'un monoester de glycérol et d'un acide carboxylique en C12-C22 contenant 110 à 500 unités d'oxyde d'éthylène dans la molécule selon la formule suivante (I) 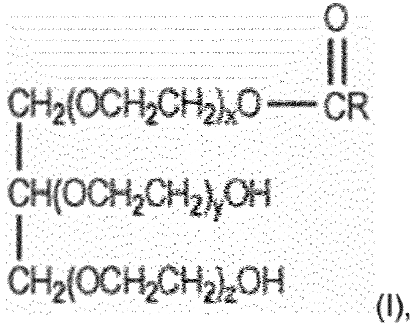 la somme des indices x + y + z étant un nombre compris dans la plage de 110 à 500 et R étant un radical alkyle contenant 11 à 21 atomes de carbone, lequel peut être saturé ou insaturé, linéaire ou ramifié, toutes les quantités se rapportant au poids de la composition d'oxydation.

2. Composition selon la revendication 1, **caractérisée en ce que** l'éther de polyéthylèneglycol selon la formule (I) est choisi parmi les composés dans lesquels la somme des indices x + y + z représente un nombre compris dans la plage de 120 à 450, de préférence un nombre compris dans la plage de 150 à 300, de manière particulièrement préférée un nombre compris dans la plage de 200 et 250.

3. Composition selon la revendication 1 ou 2, **caractérisée en ce que** l'éther de polyéthylèneglycol selon la formule (I) est choisi parmi les composés dans lesquels R représente un radical alkyle linéaire contenant 15 à 17 atomes de carbone, de préférence saturé, l'éther de polyéthylèneglycol selon la formule (I) étant de préférence choisi dans le groupe constitué de monopalmitate de glycéryle PEG-200, monostéarate de glycéryle PEG-200, monopalmitate de glycéryle PEG-120, monostéarate de glycéryle PEG-120, monopalmitate de glycéryle PEG-150, monostéarate de glycéryle PEG-150, monopalmitate de glycéryle PEG-300, monostéarate de glycéryle PEG-300, monopalmate de glycéryle PEG-200, palmate de glycéryle hydrogéné PEG-200 ainsi que leurs mélanges, choisi de manière particulièrement préférée dans le groupe palmate de glycéryle hydrogéné PEG-200.

4. Composition selon l'une des revendications 1 à 3, **caractérisée en ce qu'**au moins un éther de polyéthylèneglycol selon la formule (I) est contenu dans une quantité totale de 0,05 à 3 % en poids, de préférence de 0,1 à 2 % en poids, de manière particulièrement préférée de 0,2 à 0,7 % en poids, dans chaque cas par rapport au poids de la composition d'oxydation.

5. Composition selon l'une des revendications 1 à 4, **caractérisée en ce qu'**au moins un éther de polyéthylèneglycol d'un monoester de glycérol et d'un acide carboxylique en C8-C22 contenant 5 à 10 unités d'oxyde d'éthylène dans la molécule selon la formule suivante (II), 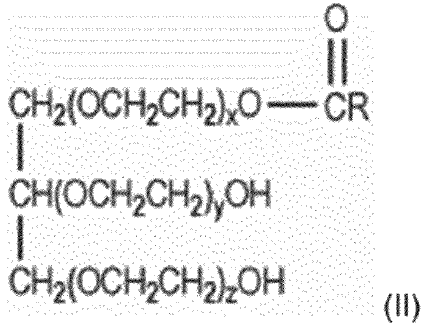 la somme des indices x + y + z étant un nombre compris dans la plage de 5 à 10 et R étant un radical alkyle contenant 7 à 21 atomes de carbone, lequel peut être saturé ou insaturé, linéaire ou ramifié.

6. Composition selon l'une des revendications 1 à 5, **caractérisée en ce que** l'éther de polyéthylèneglycol selon la formule (II) est choisi parmi des composés dans lesquels la somme des indices x + y + z est un nombre compris dans la plage de 5 à 10, de préférence un nombre compris dans la plage de 7 à 9, et R est un radical alkyle linéaire contenant 7 à 17 atomes de carbone, de préférence saturé, l'éther de polyéthylèneglycol selon la formule (II) étant de préférence choisi dans le groupe constitué de monolaurate de glycéryle PEG-7, monomyristate de glycéryle PEG-7, monopalmitate de glycéryle PEG-7, monostéarate de glycéryle PEG-7, monocaprylate de glycéryle PEG-7, monocaprate de glycéryle PEG-7, monolaurate de glycéryle PEG-10, monomyristate de glycéryle PEG-10, monopalmitate de glycéryle PEG-10, monostéarate de glycéryle PEG-10, monocaprylate de glycéryle PEG-10, monocaprate de glycéryle PEG-10, monolaurate de glycéryle PEG-5, monomyristate de glycéryle PEG-5, monopalmitate de glycéryle PEG-5, monostéarate de glycéryle PEG-5, monocaprylate de glycéryle PEG-5, monocaprate de glycéryle PEG-5, cocoate de glycéryle PEG-7 ainsi que leurs mélanges, choisi de manière particulièrement préférée dans le groupe cocoate de glycéryle PEG-7.

7. Composition selon l'une des revendications 1 à 6, **caractérisée en ce qu'**au moins un éther de polyéthylèneglycol selon la formule (II) est contenu dans une quantité totale de 0,03 à 3 % en poids, de préférence de 0,05 à 2 % en poids, de manière particulièrement préférée de 0,2 à 1,5 % en poids, de manière préférée entre toutes de 0,5 à 1,0 % en poids, dans chaque cas par rapport au poids de la composition d'oxydation.

8. Composition selon l'une des revendications 1 à 7, **caractérisée en ce qu'**elle contient au moins un 1-alcanol linéaire saturé contenant 12 à 30 atomes de carbone, de préférence en une quantité totale de 2,7 à 6 % en poids, de manière particulièrement préférée de 3,0 à 5,0 % en poids, dans chaque cas par rapport au poids de la composition d'oxydation.

9. Composition selon l'une des revendications 1 à 8, **caractérisée en ce qu'**au moins un éther de polyéthylèneglycol d'un alcanol linéaire saturé en C12-C22 contenant 15 à 30 unités d'oxyde d'éthylène est présent dans la molécule, de préférence en une quantité totale de 0,1 à 2 % en poids, de manière particulièrement préférée de 0,5 à 5,0 % en poids, dans chaque cas par rapport au poids de la composition d'oxydation.

10. Composition selon l'une des revendications 1 à 9, **caractérisée en ce qu'**elle contient au moins une huile, de préférence choisie parmi les huiles minérales et les huiles de paraffine, de préférence en une quantité totale de 0,1 à 5 % en poids, de manière particulièrement préférée de 0,2 à 3 % en poids, de manière préférée entre toutes de 0,3 à 1 % en poids, dans chaque cas par rapport au poids de la composition d'oxydation.

11. Composition selon l'une des revendications 1 à 8, **caractérisée en ce qu'**elle a un pH compris dans la plage de 2,5 à 5,5 et une viscosité comprise dans la plage de 1 500 à 3 500 mPa.s, de préférence de 2 000 à 3 000 mPa.s, mesurés à 20 °C.

12. Kit pour la modification, par oxydation, de la couleur de fibres kératiniques, comprenant deux compositions séparées (A) et (B), la composition (B) étant une composition d'oxydation selon l'une des revendications 1 à 11 et la composition (A) existant sous la forme d'une émulsion huile dans eau, contenant au moins un agent alcalinisant et au moins éventuellement un précurseur de colorant d'oxydation, et ayant un pH compris dans la plage de 8 à 11,5, mesuré à 20 °C, les compositions (A) et (B) étant de préférence présentes dans un rapport en poids (A)/(B) dans la plage de 0,33 à 3, de manière particulièrement préférée de 0,5 à 2, de manière préférée entre toutes de 1:1.

13. Kit pour la modification, par oxydation, de la couleur de fibres kératiniques selon la revendication 12, **caractérisé en ce que** la composition (A), dans chaque cas par rapport à son poids, contient 50 à 80 % en poids d'eau, en outre 5 à 2 0 % en poids, de préférence 8 à 1 5 % en poids d'au moins une matière grasse ayant un point de fusion situé dans la plage de 28 à 80 °C, de préférence choisi parmi les 1-alcanols linéaires saturés contenant 12 à 30 atomes de carbone, les esters d'alcanols en C1-C10 mono- et polyvalents et les acides alcanes en C8-C30 ainsi que leurs mélanges, en outre au moins un tensioactif non ionique et/ou anionique en une quantité totale de 1 à 8 % en poids, de préférence de 2 à 6%.

14. Procédé pour la modification, par oxydation, de la couleur de fibres kératiniques, **caractérisé par** les étapes de procédé suivantes :
fourniture d'une composition d'oxydation (B) selon l'une des revendications 1 à 11, contenant :
- 50 à 96 % en poids, de préférence 70 à 93 % en poids, de manière particulièrement préférée 80 à 90 % en poids d'eau,
- 0,5 à 20 % en poids de peroxyde d'hydrogène,
- au moins un éther de polyéthylèneglycol d'un monoester de glycérol et d'un acide carboxylique en C12-C22 contenant 110 à 500 unités d'oxyde d'éthylène dans la molécule selon la formule suivante (I) 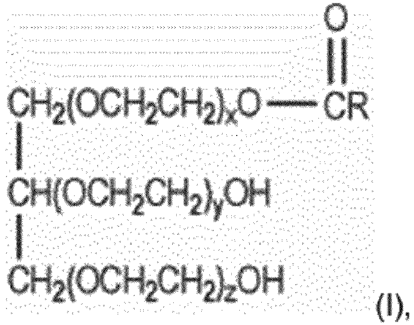 la somme des indices x + y + z étant un nombre compris dans la plage de 110 à 500 et R étant un radical alkyle contenant 11 à 21 atomes de carbone, lequel peut être saturé ou insaturé, linéaire ou ramifié, toutes les quantités se rapportant au poids de la composition d'oxydation, et fourniture d'une composition (A) présentée sous la forme d'une émulsion huile dans eau, contenant au moins un agent alcalinisant et au moins éventuellement un précurseur de colorant d'oxydation, et présentant un pH compris dans la plage de 8 à 11,5, mesuré à 20 °C, en outre de préférence, dans chaque cas par rapport à son poids, 50 à 80 % en poids d'eau, en outre de préférence 5 à 20 % en poids, de manière particulièrement préférée 8 à 15 % en poids d'au moins une matière grasse ayant un point de fusion situé dans la plage de 28 à 80 °C, de préférence choisi parmi les 1-alcanols linéaires saturés contenant 12 à 30 atomes de carbone, les esters d'alcanols en C1-C10 mono- et polyvalents et les acides alcanes en C8-C30 ainsi que leurs mélanges, en outre au moins un tensioactif non ionique et/ou anionique en une quantité totale de 1 à 8 % en poids, de préférence de 2 à 6 %, préparation d'un mélange constitué de la composition d'oxydation (B) précitée et de la combinaison (A) précitée, de préférence dans un rapport en poids (A)/(B) situé dans la plage de 0,33 à 3, de manière particulièrement préférée de 0,5 à 2, de manière préférée entre toutes de 1:1 ; puis application immédiate de l'agent prêt à l'emploi sur les fibres, maintien de l'agent sur les fibres pendant une durée de 1 à 60 minutes, puis rinçage immédiat de l'agent restant sur les fibres et séchage éventuel des fibres.

15. Procédé pour la modification par oxydation de la couleur des fibres kératiniques selon la revendication 14, **caractérisé en ce qu'**aussi bien la composition d'oxydation (B) que la composition (A) contiennent des tensioactifs cationiques, des polysaccharides et des (co)polymères d'acide (méth)acrylique, des esters d'acide (méth)acrylique, des amides d'acide (méth)acrylique ou de monomères basés sur des acides (méth)acryliques quaternisés en une quantité totale de 0 à 0,5 % en poids, de préférence de 0,05 à 0,3 % en poids, de manière particulièrement préférée de 0,08 à 0,2 % en poids, dans chaque cas par rapport au poids de la composition unique (A) ou (B).
